# EUROPEAN PATENT SPECIFICATION

(11) **EP 0 402 544 B1**
(45) Date of publication and mention of the grant of the patent: **03.05.1995**
(21) Application number: 89306016.0
(22) Date of filing: 14.06.1989
(51) Int. Cl.: C12N 15/29, C12N 1/21, A61K 35/78

(54) **Nucleotide sequence encoding plant ribosome inactivating protein**
Für Ribosomen inaktivierendes Protein aus Pflanzen kodierende Nukleotidsequenz
Séquence nucléotide codant pour une protéine végétale inactivant le ribosomes

(43) Date of publication of application: 19.12.1990
(73) Proprietor: PHARMACIA S.p.A., 20152 Milano (IT)
(72) Inventor: Lorenzetti, Rolando, I-20052 Monza (IT); Benatti, Luca, I-20090 San Maurizio, Al Lambro (Milan) (IT); Dani, Maria, I-20100 Milan (IT); Lappi, Douglas, I-20100 Milan (IT); Saccardo, Maria Beatrice, I-21047 Saronno (Varese) (IT); Soria, Marco, I-20100 Milan (IT)
(74) Representative: Woods, Geoffrey Corlett

(56) References cited:
- DE-A- 3 724 323
- GB-A- 2 216 891
- CHEMICAL ABSTRACTS, vol. 103, part 7, 19th August 1985, page 324, abstract no.51233f, Columbus, Ohio, US; D.A. LAPPI et al.: "Characterization of a Saponariaofficinalis seed ribosome-inactivating protein: immunoreactivity and sequencehomologies", & BIOCHEM.BIOPHYS. RES. COMMUN. 1985, 129(3), 934-42

## Description

This invention relates to plant ribosome inactivating proteins and, more especially, to ribosome inactivating proteins of Saponaria officinalis.

Extracts from various plants inhibit protein synthesis of animal cells. In most cases, these are ribosome inactivating proteins (RIPs). RIPs can be classified in two distinct classes. Type 2 RIPs are toxins consisting of an active A-chain linked to a cell-binding B-chain, like ricin, abrin, moddecin and viscumin. Type 1 RIPs were extracted from Phytolacca dodecandra, Phytolacca americana, Dianthus caryophyllus, Gelonium multiflorum, Momordica charantia, Saponaria officinalis, and other plants. These are single-chain proteins with the biological activity of protein synthesis inhibition but without the cell-binding activity of type 2 RIPs. Without the ability to bind cells, they are non-toxic. In 1983, Stirpe et al reported on a type 1 RIP, SO-6, with excellent stability: it is lyophilisable and can remain dry at room temperature for long periods. In addition, overnight treatment at 37°C with trypsin or chymotrypsin does not reduce RIP activity. This is an important property for a protein which must circulate through the bloodstream.

SO-6 is a protein of molecular weight 30 000. It has 40% amino acid sequence homology with the RIPs from Phytolacca americana in their N-terminal sequence, but is immunologically distinct from it and several other RIPs (Lappi et al, 1985). It is the major protein species of a family of proteins in the seed of Saponaria officinalis that are structurally highly related. They all cross react with the antisera raised against SO-6 (Lappi et al, 1986). Thorpe et al (1985) used SO-6 to make an immunotoxin that was tested in vivo against mouse AKR-A lymphoma solid tumours, with specific cytotoxicity to Thy-1.1- expressing cells both in tissue culture and animals.

Siena et al (1987) synthesised five immunotoxins by conjugating SO-6 to monoclonal antibodies that detected CD2, CD3 and CD5 T cell antigens, respectively. These immunotoxins bound to peripheral blood lymphocytes (PBL) and inhibited protein synthesis in a cell-free assay. After 2 hours at 37°C, mitogen-induced protein synthesis and cell proliferation was inhibited in a dose related manner, while unconjugated SO-6 or antibodies alone were not cytotoxic. Cytotoxicity was blocked by preincubation with unconjugated anti- CD5 but not with an irrelevant antibody, thus demonstrating that it was caused by specific binding to CD5+ cells.

Recombinant DNA methodologies have been employed to construct fused genes between a toxin and a ligand. The first example of such construction was provided by Murphy et al (1986) who fused the genes coding for a truncated diphtheria toxin fragment and for melanocyte - stimulating hormone (alpha - MSH). The toxin-hormone chimeric gene directed the expression of a fused protein that retained the ADP-ribosyltransferase activity and lipid-associating domains of diphtheria toxin. However, the diphtheria toxin receptor-binding domain was replaced with MSH sequences. The chimeric toxin was found to be toxic for MSH receptor-positive human malignant melanoma cells in culture, while not being toxic for either Chinese hamster ovary or African green monkey kidney cells, both of which lack the alpha-MSH receptor.

More recently, Williams et al (1987) have extended these earlier observations by showing that fusion of the gene encoding interleukin-2 (IL-2) to a truncated diphtheria toxin gene also results in the expression of a biologically active chimeric IL-2 toxin. The fused protein was shown to be selectively targeted to activated or to malignant T cells carrying specific surface receptors for the ligand components of the hybrid, and was internalised by receptor - mediated endocytoses following in vitro binding to the IL-2 receptor.

A cloned Pseudomonas toxin (PE), from which the cell-recognition domain was deleted by site-directed mutagenesis, was fused to Transforming Growth Factor-alpha (TGF-alpha). The chimeric protein, purified from E coli, killed cells expressing epidermal growth factor receptors and had little activity against cells with few receptors (Chaudhary et al, 1987).

We have now cloned and expressed a gene encoding the type 1 RIP SO-6 of Saponaria officinalis. Accordingly, the present invention provides a DNA sequence:

1                    24                    50
GTCACATCAATCACATTAGATCTAGTAAATCCGACCGCGGTCAATACTCATCTTTTGTG
                                        100
GATAAAATCCGAAACAACGTAAAGGATCCAAACCTGAAATACGGTGGTACCGACATAGCC
                         150
GTGATAGGCCCACCTTCTAAAGAAAAATTCCTTAGAATTAATTTCCAAAGTTCCCGAGGA
                  200
ACGGTCTCACTTGGCCTAAAACGCGATAACTTGTATGTGGTCGCGTATCTTGCAATGGAT
      250                                              300
AACACGAATGTTAATCGGGCATATTACTTCAGATCAGAAATTACTTCCGCCGAGTCAACC
                                                  350
GCCCTTTTCCCAGAGGCCACAACTGCAAATCAGAAAGCTTTAGAATACACAGAAGATTATC
                                        400
AGTCGATTGAAAAGAATGCCCAGATAACACAAGGAGATCAAAGTAGAAAAGAACTCGGGT
                         450
TGGGGATTGACTTACTTTCAACGTCCATGGAAGCAGTGAACAAGAAGGCACGTGTGGTTA
                  500
AAGACGAAGCTAGATTCCTTCTTATCGCTATTCAGATGACGGCTGAGGCAGC
               550
GCGATTTAGGTACATACAAAACTTGGTAATCAAGAACTTTCCCAACAAGTTCAACT

          600

CGGAAAACAAAGTGATTCAGTTTGAGGTTAACTGGAAAAAAATTTCTACGGCAATATA

650                                                                                    700

CGGGGATGCCAAAAACGGCGTGTTTAATAAAGATTATGATTTCGGGTTTGGAAAAGTTAG

                                                                          750

GCAGGTGAAGGACTTGCAAATGGGACTCCTTATGTATTTGGGCAAACCAAAGTCGTC

768
AAAC.

This sequence may be preceded by a signal sequence. Preferably the signal sequence is:

ATGAAGATATATGTTGTAGCCACAATAGCATGGATCCTGCTTCAATTTTCAGCTTGG
ACAACAACTGATGCG.

A sequence may end with a termination codon such as TAG after the final codon AAC. Alternatively, this codon may be absent if it is wished to obtain a fusion protein comprising the RIP fused to a ligand/haptomer capable of binding to cells.

The DNA sequence coding for RIP SO-6 can be obtained by a method which comprises:
(i) isolating mRNA from the leaves of Saponaria officinalis;
(ii) synthesising therefrom cDNA;
(iii) inserting the resulting cDNA into a cloning vector to obtain a cDNA library;
(iv) probing the cDNA library with a labelled DNA probe corresponding to a portion of the amino acid sequence of the said RIP to locate a clone comprising a said DNA sequence and
(v) optionally, isolating a DNA sequence comprising the said DNA sequence from the clone.

The mRNA is preferably extracted from the leaves or seeds of Saponaria officinalis. The cDNA can be prepared according to standard procedures. For example, synthesis of the first strand of the cDNA can be accomplished using reverse transcriptase. The second strand is typically synthesised using DNA polymerase followed by T4 polynucleotide kinase.

The cDNA is inserted into a cloning vector to obtain a cDNA library. The cloning vector may be a plasmid or phage viral vector. If the cloning vector is a plasmid, it may be a naturally-occurring plasmid or preferably a composite plasmid derived from fragments of other plasmids. The plasmid may contain a promoter sequence to improve the expression of the RIP gene. The cDNA library can be amplified in a suitable host, e.g. E. coli.

The library is probed by one or more labelled DNA sequence corresponding to a portion of the amino acid sequence of the RIP whose gene is being sought. The DNA probe sequences can be deduced from the amino acid sequence of the RIP if the DNA sequence of the RIP is not known. The probe sequences may correspond to the C-terminal or N-terminal portion of the RIP. The sequences may be up to 120 bp long. A radioactive label can be employed.

In this way, one or more clones in the cDNA library may be located which comprise a DNA sequence coding for the desired RIP. This DNA sequence, or a longer DNA sequence comprising it, may be isolated using appropriate restriction endonucleases. The DNA sequence can then be cloned and expressed as desired.

In order to obtain RIP SO-6, a DNA sequence according to the invention is incorporated in an expression vector which is capable of expressing the RIP in a transformed host. The DNA sequence is provided in the expression vector operably linked to expression control elements, in particular a promoter. The vector typically has an origin of replication and a phenotypic marker. The DNA sequence to be expressed is provided between translational start and stop signals. The vector is typically a plasmid.

The DNA sequence may also comprise a further sequence encoding a ligand/haptomer capable of binding to cells, for example when it is wished to express an immunotoxin. The ligand/haptomer may be a monoclonal antibody which is specific for a tumor cell antigen or a F(ab′)₂ fragment thereof. It may be an anti-T cell antibody. Other ligands/haptomers may be hormonal proteins which bind to specific receptor sites. The ligand/haptomer may alternatively be chemically conjugated to a RIP produced according to the invention to obtain an immunotoxin.

A host transformed with an expression vector according to the invention can be cultured to obtain the desired RIP or, when the DNA sequence incorporated in the expression vector further comprises a sequence encoding a ligand portion of an immunotoxin, the desired immunotoxin. An appropriate host may be employed, for example a plant or animal cell or a microoganism. A bacterial host such as E. coli may be employed. The RIP or immunotoxin thus expressed can be isolated from the culture by standard methods.

An immunotoxin, expressed as such or obtained by conjugating a ligand capable of binding to cells to expressed RIP, is generally formulated for administration with a pharmaceutically acceptable carrier or diluent. The immunotoxin may be administered by injection. In this event, it may be formulated with a sterile, pyrogen-free liquid such as Water for Injections or physiological saline.

The following Example illustrates the present invention. In the accompaning drawings:
Figure 1 shows the amino acid sequence of five CNBr fragments of SO-6,
Figure 2 is a restriction map of the EcoRI insert of clone pBL6,
Figure 3 shows the DNA sequence and the predicted corresponding amino acid sequence of the EcoRI insert of clone pBL6, and
Figure 4 compares the CNBr fragments of SO-6 with the predicted amino acid sequence.

### Example

### 1. Amino acid sequence of CNBr fragments of the Saponaria officinalis SO-6 RIP

### SO-6 purification

SO-6 was prepared as previously described by Stirpe et al (1983).

### CNBr cleavage and fragment purification

Ten milligrams of purified SO-6 were dissolved in 300 ul of 70% formic acid. About 30 mg of CNBr were added, and after 14-18 hours at room temperature the reaction mixture was diluted to 3 ml with deionised water and lyophilised. The resulting peptides were purified by gel filtration on Sephadex G-100 and hydrophobic reverse phase HPLC.

### Amino acid and sequence analysis

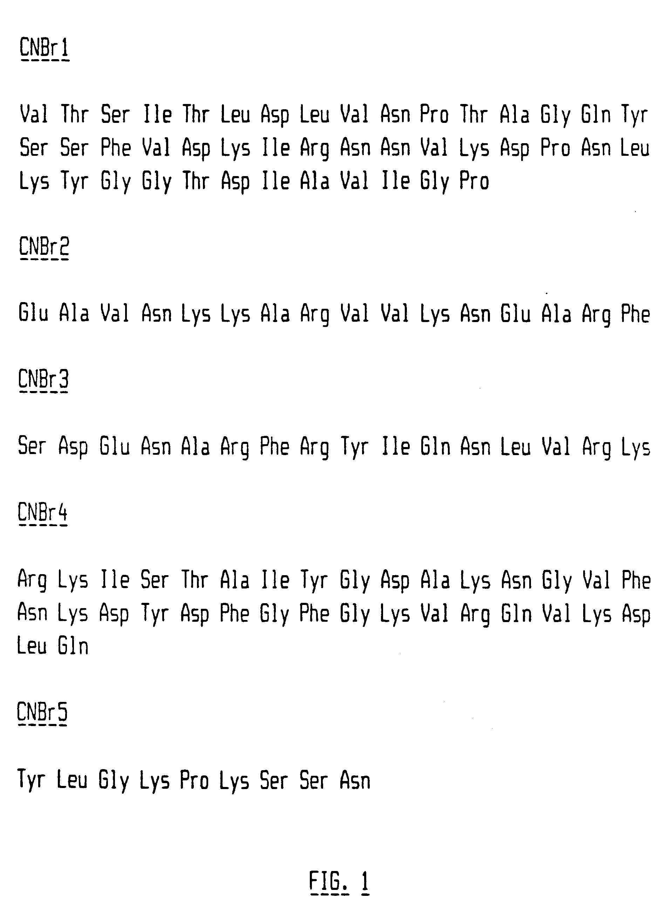

Amino-acid analysis was carried out with the PICO-TAG analyser (Waters). Hydrolysis was performed in vacuo for 24 hours at 105°C in constant boiling HCl containing 1% phenol. Sequence analysis was performed by a gas phase sequenator (Applied Biosystems). Figure 1 shows the amino acid sequence of five CNBr fragments of SO-6. CNBr5 in the C-terminal CNBr fragment was identified by the lack of homoserine in the amino acid analysis.

### 2. cDNA Library Construction

### RNA extraction

10-20 g of frozen leaves were homogenised with an Ultra Turrax for 5 minutes at top speed in 60 ml of 4.2 M guanidine-thiocyanate, 25 mM Na citrate, 5% Sarcosyl, 0.7 mM Mercaptoethanol, 0.01% antifoam, pH7. The homogenised slurry was filtered through a sterile cheese cloth and centrifuged at 5 000 rpm for 10 minutes at 4°C. The supernatant was layered over 5.7 M CsCl, 25 mM Na acetate pH 5.4, 0.1 M EDTA, and centrifuged at 31 000 rpm in a SW 40 rotor for 20 hours at 20°C. The total RNA pellet was washed with cold 70% ethanol and resuspended in a few millilitres of 10 mM Tris-HCl, 5 mM EDTA pH 7, extracted once with chloroform, salt adjusted to 0.3 M Na-acetate and precipitated with 2.5 volumes of cold ethanol. After 1 to several hours at -80°C the RNA was centrifuged at 10 000 rpm for 1 hour in a Sorvall centrifuge. The pellet was washed with cold 70% ethanol and resuspended in binding buffer.

Approximately 1 mg of total RNA was recovered from each gram of leaves.

Poly(A)+ RNA was isolated by affinity chromatography on oligo (dT)- cellulose (Aviv and Leder, 1972).

Approximately 20 ug of Poly(A)+ RNA were recovered from 1 mg of total RNA. The length of the Poly(A)+ RNA was verified on a 1% agarose gel containing formaldehyde. The size of the RNA ranged from several Kb to a few hundred bases.

### First strand cDNA synthesis

The first strand cDNA synthesis was carried out for 40 minutes at 42°C in 50 ul of: 50 mM Tris-HCl buffer pH 8.5, 40 mM KCl, 10 mM MgCl₂, 0.4 mM DTT; 1 mM dATP; 1 mM dGTP; 1 mM dTTP; 0.5 mM dCTP; 0.1 mg/ml oligo (dT)₁₂₋₁₈; 25 U Human placental ribonuclease inhibitor; 20 uCi [alpha-³²P] dCTP 3000 Ci/mmol, 5 ug poly (A)+ RNA and 40 units of AMV reverse transcriptase.

### Second strand cDNA synthesis

To the first strand cDNA reaction mixture, 93.5 ul of second strand buffer (100 mM HEPES pH 6.9, 100 mM KCl, 10 mM MgCl₂); 20 uCi [alpha-³²P] dCTP 3000 Ci/mmol; 4 U E coli ribonuclease H; 115 U E coli DNA polymerase I were added in a final volume of 250 ul.

The reaction mixture was incubated for 1 hour at 12°C, 1 hour at 22°C and 10 minutes at 70°C. After the mixture was cooled down on ice, 10 U of T4 DNA polymerase were added, and the mixture incubated at 37°C for 10 minutes.

### Addition of EcoRI linkers and EcoRI digestion

The cDNA was purified by phenol: chloroform (1:1) extraction and ethanol precipitation. 1 mg of phosphorylated EcoRI linkers were added to the double stranded cDNA preparation in a 20 ul reaction containing 66 mM Tris-HCl pH 7.5; 5 mM MgCl₂, 5 mM dithiothreitol, 1 mM ATP (ligation buffer) in the presence of 1 U of T4 DNA ligase. The mixture was incubated overnight at 12°C.

After addition of NaCl to 100 mM final concentration, spermidine to 2.5 mM and 30 units of ECO RI in a final volume of 100 ul, the mixture was incubated for 2 hours at 37°C.

The cDNA was purified from the non-incorporated linkers passing it through a Sepharose 4B column in 0.3 M NaCl, 10 mM Tris-HCl pH8, 1 mM EDTA. cDNA fractions ranging in size between 8 Kb and 0.5 Kb were pooled and ethanol precipitated.

### Ligation of cDNA to λgt 10 arms and in vitro packaging

0.5 ug of λgt 10 arms were ligated to cDNA with 2.5 units of T4 DNA ligase in ligation buffer with a final volume of 5 ul and incubated overnight at 15°C.

The DNA was then ethanol precipitated and carefully resuspended in 2.5 ul of 10 mM Tris-HCl pH 7.5, 1 mM EDTA for in vitro packaging with an in vitro packaging mixture (Amersham).

The library obtained was amplified using E coli NM514 host. The number of independent clones obtained was 3.3 x 10¹⁰ with a background of non-recombinant phases of 36%.

### 3. cDNA library screening

### Synthesis of oligonucleotides

a - Synthesis of a 111 bp long oligonucleotide. This long oligonucleotide corresponded to the first 37 amino acids at the NH₂ terminal of SO-6 RIP. The codon usage was chosen based on the codon frequency of the seed storage proteins sequenced so far as deduced from sequence databases (GenBank). The long oligonucleotide was synthesised using an Applied Biosystems Inc Mod 380B automatic DNA synthetiser, purified by reverse-phase HPLC and assembled in double stranded form using 8 different oligonucleotides (19 to 28 bases long).
   The oligonucleotides were ligated together and the resulting double stranded oligonucleotide was inserted into the SmaI site of M13mp8 and sequenced in order to verify the nucleotide sequence.
b - Synthesis of short oligonucleotides.
   A mixture of 16 short (21 bases) oligonucleotides, corresponding to CNBr fragment of the C-termini of SO-6, was synthesised using the same Applied Biosystem Inc synthesiser described above.

### Labelling of oligonucleotides

### a - 111 bp "long" oligonucleotide.

This oligonucleotide, inserted into the ss DNA phage M13mp8 as described, was labelled by DNA polymerase after annealing to a primer complementary to the M13 sequence adjacent to the oligonucleotide. About 6 ug of primer were annealed to 5 ug of M13mp8-111 oligonucleotide in 7 mM Tris-HCl pH 7.5, 7 mM MgCl₂, 50 mM NaCl, 10 mM DTT, 0.1 mM EDTA pH 8.0 (1 x klenow buffer) in a volume of 30 ul for 1 hour at 60°C 50 ul of [alpha - ³²P] dCTP 3.000 Ci/mmol, dCTP, dTTP, dATP at a final 50 uM concentration and 5 units of DNA poymerase (klenow fragment) were added in a final volume of 45 ul.

After incubation at room temperature for 15 minutes, 1 ul of 1 mM dCTP was added and the mixture incubated again for 15 minutes at RT.

The enzyme was inactivated at 70°C for 10 minutes. After addition of 1.3 ul of 5M NaCl and 20 units of each Eco RI and Bam HI, the mixture was incubated for 2 hours at 37°C, in order to cut the 111 bp long oligonucleotide out of the phage vector. The oligonucleotide was then separated from the vector on a 3.5% PAGE and eluted overnight in H₂O at 37°C. The specific activity was about 5 x 10⁸ DPM/ug DNA.

### b - "Short" mixed oligonucleotides.

The mixture of short (21 bp) oligonucleotides was end-labelled using T4 polynucleotide kinase as described by Davies et al, 1986.

### Plaque hybridisation using the oligonucleotides as probes

### a - Screening of the cDNA library with the 111 bp long oligonucleotide.

About 200 000 phages were plated on a lawn of E coli NM514 cells. After an overnight growth at 37°C the recombinant phages were transferred in duplicate nitrocellulose filters, their DNA was denatured, neutralised and baked under vacuum at 80°C for 2 hours.

The filters were prehybridised in 6x SSC, 5x Denhardt's, 0.1% SDS, 100 ug/ml salmon sperm DNA at 50°C for 2 hours.

The filters were then hybridised overnight at 50°C in the same mixture with the addition of 1 x 10⁶ cpm/ml of labelled probe (probe a). The filters were washed in 0.1 x SSC, 0.1% SDS at 60°C and autoradiographed.

Positive phage phaques were isolated and screened again twice in order to isolate single clones.

### b - Screening of the positive clones with the oligonucleotide mixture (probe b).

The clones that hybridised to the 111 bp probe were plated and screened with the labelled "short" oligonucleotide mixture.

The filters were prehybridised in 6x SSC, 5x Denhardt's, 0.1% SDS, 100 ug/ml salmon sperm DNA at 42°C.

The filters were then hybridised overnight at 42°C after addition of the labelled oligonucleotide mixture.

The filters were then washed in 6x SSC, 0.1% at 45°C and autoradiographed. One of the clones positive to both probes was isolated and sequenced.

### DNA sequencing

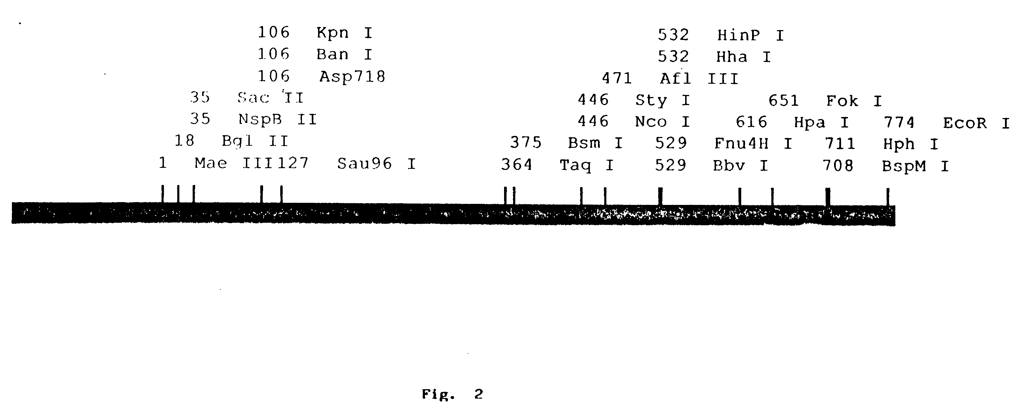

The DNA of the positive clone pBL6 was isolated by the Promega LambdaSorb phage adsorbent method, the insert was removed with EcoRI and ligated to the EcoRI site of M13mp8 in both directions. The restriction endonuclease map of pBL6 clone is shown in Figure 2. Sequencing was carried out by the Sanger procedure. Both strands of the gene were sequenced, revealing an open reading frame coding for a protein of 280 amino acids.

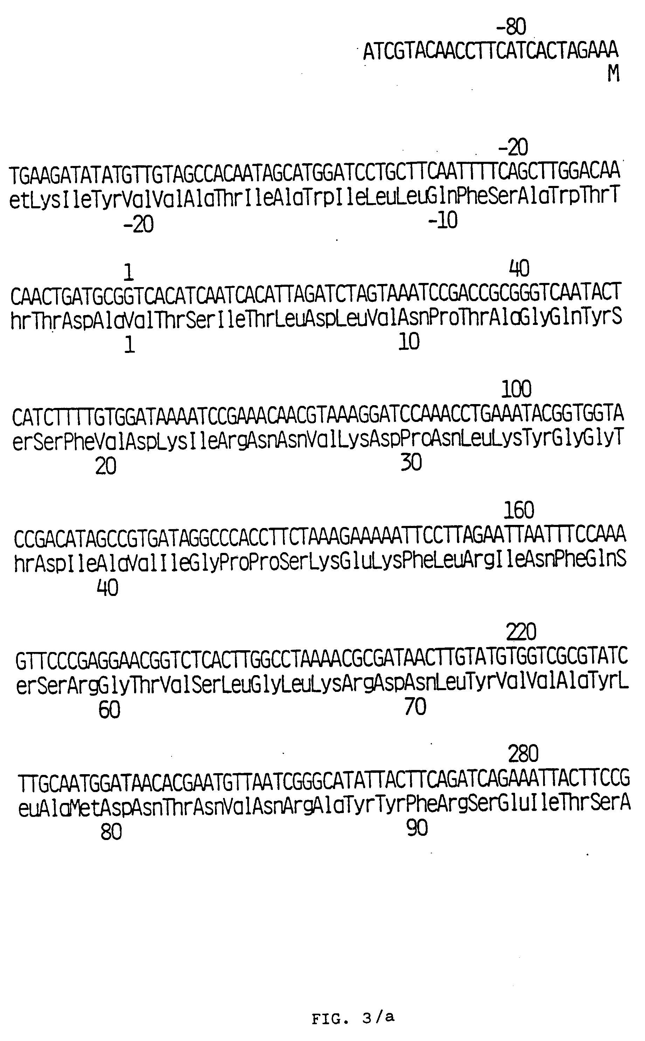

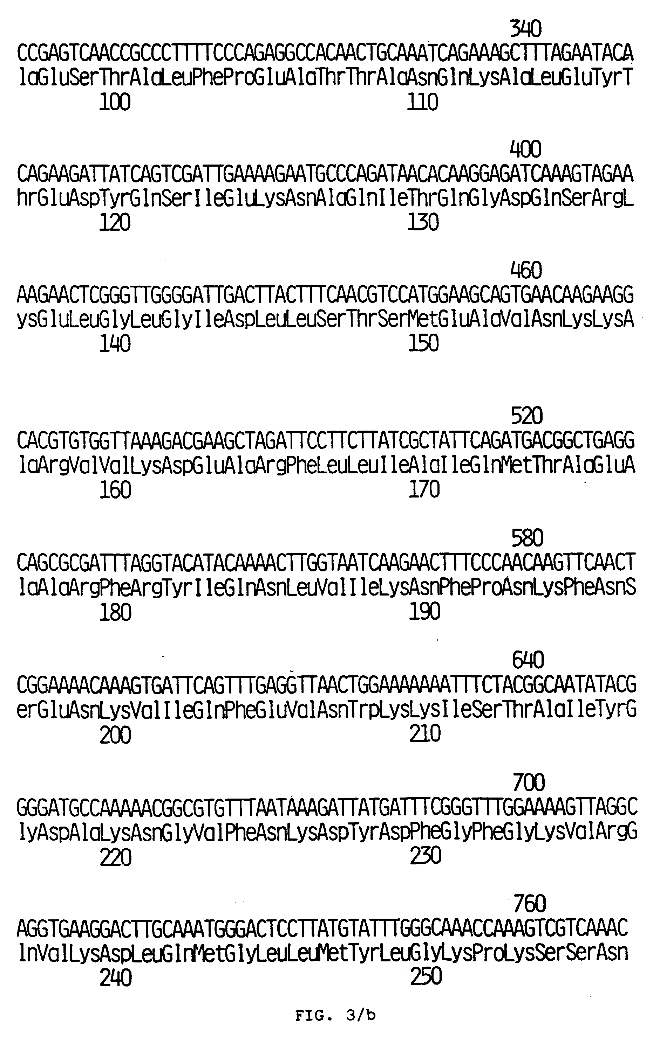

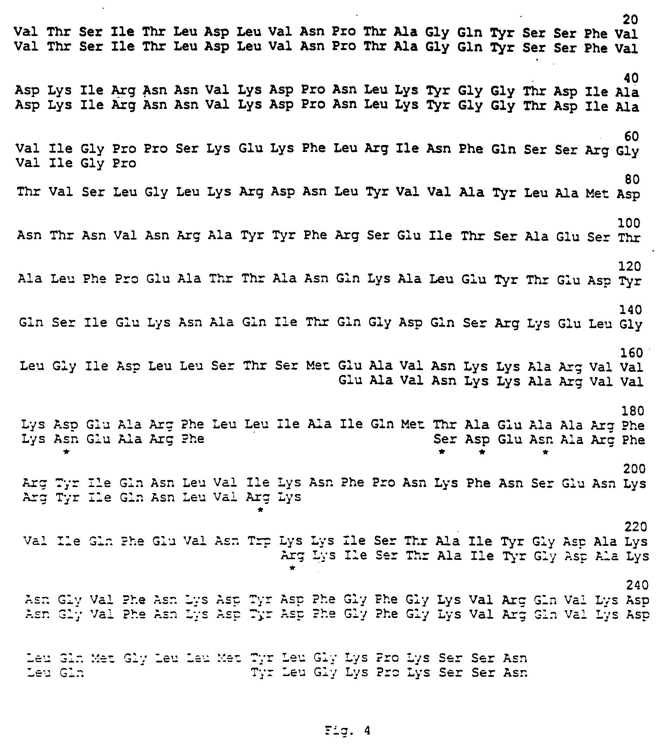

Comparing the published N-terminal amino acid sequence of SO-6 as reported by Lappi et al (1985) to that of clone pBL6, we could predict the amino acid sequence start point of the mature protein encoded by our clone (Figure 3). The translation initiation site was assigned to the methionine codon d(ATG) present at nucleotide residues -72 to -70. The sequence also predicts an N-terminal extension of 24 amino acids for the signal peptide. This data is in agreement with the length (24 amino acids) of the ricin signal peptide. However, comparison of the CNBr fragments of SO-6 to the predicted amino acid sequence of clone pBL6 showed six differences at single amino acid residues (Figure 4). This could be due to mistakes in amino acid sequencing, or to sequence heterogeneities in the RIPs purified from Saponaria officinalis (Stirpe et al, 1983).

### 4. Expression in E. coli

Starting from the SO-6 gene carried by clone pBL6, the 900 bp EcoRI fragment (Fig. 2) was subcloned into the EcoRI site of the vector pUC 8 (Amersham, U.K.). The plasmid thus obtained was then cut out with Bgl II and Pst I, allowing retrieval of a fragment containing the SO-6 gene but lacking the 5′ region encoding the signal peptide and the first 6 amino acid residues (Fig. 2 and 3). At the 3′ end of the fragment the SO-6 cDNA was followed by an extra piece of DNA deriving from the pUC 8 polylinker between the EcoRI and the Pst I site.

Ligation of the Bgl II-Pst I fragment carrying the gene for SO-6 to the expression vector pUEX3 (Bressan and Stanley, 1987) cut with BamHI and Pst I, resulted in an in-frame fusion between the gene coding for beta-galactosidase (Stanley and Luzio, 1984) and teh gene containing most of the coding sequence for SO-6. Downstream from the SO-6 gene, translation termination was provided by stop codons present in the pUEX3 vector.

The recombinant plasmid thus obtained was transformed into the bacterial host E. coli DM105 (Amersham, UK). The hybrid gene, under the control of the lambda R_{R} promoter, was expressed essentially in accordance with Zabeau and Stanley (1982). Bacteria were grown at 30°C to OD₆₀₀ 0.9. The temperature was quickly raised to 42°C by addition of an equal volume of broth pre-heated at 54°C. Cultures were incubated at 42°C for 2 hours before harvest. Total cell lysates were loaded on polyacylamide slab gels (at the appropriate concentration) using the Laemmli method. For the immunoblotting, the gels were transferred on nitrocellulose filters using a transblot apparatus (Bio-Rad) at 0.2 A for 4 hours at 4°C in 25 mM Tris Base, 192 mM Glycine, 20% Methanol. After transfer the filters were washed with distilled water and then incubated 1 hour with PBS + 3% BSA with gentle shaking. Filters were washed again with distilled water and incubated with an anti-SO-6 antiserum raised in rabbits diluted 1:250 with PBS, and incubated 1 hour at room temperature. After 2 washes with PBS and PBS + 0.05% Tween 20, the filters were incubated with an anti rabbit IgG raised in goat conjugated with horseradish peroxidase (Bio-Rad) diluted 1:7500 for 1 hour at room temperature. After two more washes, filters were stained with 4-chloro-1-napthol.

Using these procedures, the molecular weight of the hybrid beta galactosidase - SO-6 protein was found to be, as expected, 145 Kd on SDS-PAGE. The band migrating at this position on the gel was specifically recognized by an anti-SO-6 antiserum by the immunoblot procedure described above.

The following method was used for the purificatiuon of the hybrid protein. Bacterial pellets from 100 ml culture were resuspended in 3-5 ml of buffer A (50 mM Tris/HCl pH 7.4, 170 mM NaCl, 2.5 mg/ml lysozyme), incubated 30 min in ice and sonicated 5 times for 20 sec on ice. After centrifugation (10,000 rpm, 40 min at 4°C in a Sorvall SS34 rotor), the pellet was resuspended in 10 ml of buffer B (7 M urea, 10 mM Tris-HCl pH 7.4, 1 mM EDTA) and left at room temperature for 30 min. The suspension was centrifuged as above and the supernatant was dialysed extensively at 4°C against 2 l of 50 mM Tris HCl pH 7.4.

Purification was achieved by affinity chromatography on p-aminophenyl thiogalactoside-Sepharose as described by Ullmann (1984). SDS-PAGE of the purified material was run in parallel to the unpurified material as described above, and revealed a prominent band of the expected molecular weight that was recognized by the specific anti-SO-6 antiserum after immunoblotting by the procedure already described. The purified recombinant protein corresponded in migration to the hybrid beta galactosidase-SO6 present in extracts of the induced E. coli strain.

### REFERENCES:

Aviv and Leder, Proc. Natl. Acad. Sci. USA, 69 (1972) 1408.

Bressan and Stanley, Nucleic Acid Research 15, 10056, 1987.

Chaudhary et al, Proc. Natl. Acad. Sci. USA, 84 (1987) 4538-4542.

Davis et al, Basic methods in molecular biology, 1986, Elsevier Science Publishing Co., Inc.

Lappi et al, Biochem. Biophys. Res. Comm., 129 (1985) 934-942.

Lappi et al, Abs. 15th annual UCLA Symposia on Molecular and Cellular Biology, Park Utah, February 22, March 23, 1986.

Murphy et al, Proc. Natl. Acad. Sci., USA, 83 (1986) 8258-8262.

Siena et al, Abst. American Assoc. Cancer Res., Atlanta, GA., 1987.

Stanley and Luzio, EMBO Journal 3, 1429-1434, 1984.

Stirpe et al, Biochem. J., 216 (1983) 617-625.

Thorpe et al, J. N. C. I., 75 (1985) 151-159.

Ullmann, Gene 29, 27-31, 1984.

Williams et al, Abst. Int. Symp. "Protein Engineering'87", Oxford, UK, 5-8 April, 1987.

Zabeau and Stanley, EMBO Journal 1, 1217-1224, 1982.

## Claims (Claims for the following Contracting State(s): BE, FR, DE, NL, SE, CH)

1. A DNA sequence which encodes ribosomal inactivating protein (RIP) SO-6 and which is:

1                    24                    50

GTCACATCAATCACATTAGATCTAGTAAATCCGACCGCGGTCAATACTCATCTTTTGTG

                                                  100

GATAAAATCCGAAACAACGTAAAGGATCCAAACCTGAAATACGGTGGTACCGACATAGCC

                         150

GTGATAGGCCCACCTTCTAAAGAAAAATTCCTTAGAATTAATTTCCAAAGTTCCCGAGGA

                200

ACGGTCTCACTTGGCCTAAAACGCGATAACTTGTATGTGGTCGCGTATCTTGCAATGGAT

     250                                                  300

AACACGAATGTTAATCGGGCATATTACTTCAGATCAGAAATTACTTCCGCCGAGTCAACC

                                                       350

GCCCTTTTCCCAGAGGCCACAACTGCAAATCAGAAAGCTTTAGAATACACAGAAGATTAT

                                            400

CAGTCGATTGAAAAGAATGCCCAGATAACACAAGGAGATCAAAGTAGAAAAGAACTCGGG

                         450

TTGGGGATTGACTTACTTTCAACGTCCATGGAAGCAGTGAACAAGAAGGCACGTGTGGTT

                500

AAAGACGAAGCTAGATTCCTTCTTATCGCTATTCAGATGACGGCTG

                    550

AGGCAGCGCGATTTAGGTACATACAAAACTTGGTAATCAAGAACTTTCCCAACAAGTTCAA

          600

CTCGGAAAACAAAGTGATTCAGTTTGAGGTTAACTGGAAAAAAATTTCTACGGCAATATA

650                                                  700

CGGGGATGCCAAAAACGGCGTGTTTAATAAAGATTATGATTTCGGGTTTGGAAAAGTTAG
750
GCAGGTGAAGGACTTGCAAATGGGACTCCTTATGTATTTGGGCAAACCAAAGTCGTC
768
AAAC.

2. A DNA sequence composed of the DNA sequence of claim 1 immediately preceded by the following signal sequence:

ATGAAGATATATGTTGTAGCCACAATAGCATGGATCCTGCTTCAATTTTCAGCTTGG
ACAACAACTGATGCG.

3. A DNA sequence as shown in Figure 3 of the accompanying drawings.

4. A cloning vector comprising a DNA sequence as claimed in any one of the preceding claims.

5. A vector according to claim 4 which is a plasmid.

6. A vector according to claim 5 which is a phage viral vector.

7. An expression vector which incorporates a DNA sequence as claimed in any one of claims 1 to 3 and which is capable, in a transformed host, of expressing the said RIP.

8. A vector according to claim 7, which is a plasmid.

9. A vector according to claim 7 or 8 which is capable, in a transformed host, of expressing a conjugate comprising the said RIP conjugated to a ligand capable of binding to cells.

10. A host transformed with a vector as claimed in any one of claims 7 to 9.

11. A process for the preparation of RIP SO-6 or a conjugate comprising the said RIP conjugated to a ligand capable of binding to cells, which process comprises culturing a transformed host as claimed in claim 10 and isolating the said RIP or conjugate thus produced.

12. A pharmaceutical composition comprising a conjugate which has been produced by a process as claimed in claim 11 and a pharmaceutically acceptable carrier or diluent.

## Claims (Claims for the following Contracting State(s): ES)

1. A process for the preparation of ribosomal inactivating protein (RIP) SO-6 or a conjugate comprising said RIP conjugated to a ligand capable of binding to cells, which process comprises culturing a host transformed with a vector capable of expressing said RIP or said conjugate, comprising the DNA sequence 
1
24
50
GTCACATCAATCACATTAGATCTAGTAAATCCGACCGCGGTCAATACTCATCTTTTGTG
100
GATAAAATCCGAAACAACGTAAAGGATCCAAACCTGAAATACGGTGGTACCGACATAGCC
150
GTGATAGGCCCACCTTCTAAAGAAAAATTCCTTAGAATTAATTTCCAAAGTTCCCGAGGA
200
ACGGTCTCACTTGGCCTAAAACGCGATAACTTGTATGTGGTCGCGTATCTTGCAATGGAT
250
300
AACACGAATGTTAATCGGGCATATTACTTCAGATCAGAAATTACTTCCGCCGAGTCAACC
350
GCCCTTTTCCCAGAGGCCACAACTGCAAATCAGAAAGCTTTAGAATACACAGAAGATTAT
400
CAGTCGATTGAAAAGAATGCCCAGATAACACAAGGAGATCAAAGTAGAAAAGAACTCGGG
450
TTGGGGATTGACTTACTTTCAACGTCCATGGAAGCAGTGAACAAGAAGGCACGTGTGGTT
500
AAAGACGAAGCTAGATTCCTTCTTATCGCTATTCAGATGACGGCTG
550
AGGCAGCGCGATTTAGGTACATACAAAACTTGGTAATCAAGA[?]ACTTTCCCAACAAGTTCAA
600
CTCGGAAAACAAAGTGATTCAGTTTGAGGTTAACTGGAAAAAATTTCTACGGCAATATA
650
700
CGGGGATGCCAAAAACGGCGTGTTTAATAAAGATTATGATTTCGGGTTTGGAAAAGTTAG
750
GCAGGTGAAGGACTTGCAAATGGGACTCCTTATGTATTTGGGCAAACCAAAGTCGTC
768
AAAC
 and isolating said RIP or conjugate thus produced.

2. A process according to claim 1, wherein said DNA sequence is immediately preceded by the following signal sequence:

ATGAAGATATATGTTGTAGCCACAATAGCATGGATCCTGCTTCAATTTTCAGCTTGG
ACAACAACTGATGCG.

3. A process according to claim 1, wherein the vector Comprises the DNA sequence shown in Figure 3 of the accompanying drawings.

4. A process according to claim 1, 2 or 3, wherein said vector is a plasmid.

5. A process according to claim 1, 2 or 3, wherein said vector is a phage viral vector.

6. A process according to any one of the preceding claims, which further comprises formulating said conjugate with a pharmaceutically acceptable carrier or diluent to produce a pharmaceutically acceptable composition.

7. A process for producing a DNA molecule comprising the DNA sequence as defined in claim 1, which process comprises:
(i) isolating mRNA from the leaves of Saponaria officinalis;
(ii) synthesising therefrom cDNA;
(iii) inserting the resulting cDNA into a cloning vector to obtain a cDNA library;
(iv) probing the cDNA library with a labelled DNA probe corresponding to a portion of the amino acid sequence of the said RIP to locate a clone comprising a said DNA sequence and
(v) isolating a DNA sequence comprising the said DNA sequence from the clone.

## Patentansprüche (Patentansprüche für folgende(n) Vertragsstaat(en): BE, FR, DE, NL, SE, CH)

1. DNA-Sequenz, die für das ribosomale inaktivierende Protein (RIP) SO-6 kodiert, und welche ist:

1
24
50
GTCACATCAATCACATTAGATCTAGTAAATCCGACCGCGGTCAATACTCATCTTTTGTG
100
GATAAAATCCGAAACAACGTAAAGGATCCAAACCTGAAATACGGTGGTACCGACATAGCC
150
GTGATAGGCCCACCTTCTAAAGAAAAATTCCTTAGAATTAATTTCCAAAGTTCCCGAGGA
200
ACGGTCTCACTTGGCCTAAAACGCGATAACTTGTATGTGGTCGCGTATCTTGCAATGGAT
250
300
AACACGAATGTTAATCGGGCATATTACTTCAGATCAGAAATTACTTCCGCCGAGTCAACC
350
GCCCTTTTCCCAGAGGCCACAACTGCAAATCAGAAAGCTTTAGAATACACAGAAGATTAT
400
CAGTCGATTGAAAAGAATGCCCAGATAACACAAGGAGATCAAAGTAGAAAAGAACTCGGG
450
TTGGGGATTGACTTACTTTCAACGTCCATGGAAGCAGTGAACAAGAAGGCACGTGTGGTT
500

AAAGACGAAGCTAGATTCCTTCTTATCGCTATTCAGATGACGGCTG
550
AGGCAGCGCGATTTAGGTACATACAAAACTTGGTAATCAAGAACTTCCCAACAAGTTCAA
600
CTCGGAAAACAAAGTGATTCAGTTTGAGGTTAACTGGAAAAAATTTCTACGGCAATATA
650 700
CGGGGATGCCAAAAACGGCGTGTTTAATAAAGATTATGATTTCGGGTTTGGAAAAGTTAG
750
GCAGGTGAAGGACTTGCAAATGGGACTCCTTATGTATTTGGGCAAACCAAAGTCGTC
768
AAAC.

2. DNA-Sequenz, zusammengesetzt aus der DNA-Sequenz gemäß Anspruch 1, der unmittelbar die folgende Signalsequenz vorausgeht:

ATGAAGATATATGTTGTAGCCACAATAGCATGGATCCTGCTTCAATTTTCAGCTTGGACA
ACAACTGATGCG.

3. DNA-Sequenz gemäß Fig. 3 anliegenden Zeichnungen.

4. Klonierungsvektor, umfassend eine DNA-Sequenz gemäß einem der vorhergehenden Ansprüche.

5. Vektor nach Anspruch 4, der ein Plasmid ist.

6. Vektor nach Anspruch 5, der ein viraler Phagen-Vektor ist.

7. Expressionsvektor der eine DNA-Sequenz umfaßt, nach einem der Ansprüche 1 bis 3 und der zur Expression des RIPs in einem transformierten Wirt in der Lage ist.

8. Vektor nach Anspruch 7, der ein Plasmid ist.

9. Vektor nach Anspruch 7 oder 8, der zur Expression eines Konjugats, das das RIP, welches an einen zur Bindung an Zellen fähigen Liganden konjugiert ist, umfaßt, in einem transformierten Wirt in der Lage ist.

10. Wirt, transformiert mit einem Vektor nach einem der Ansprüche 7 bis 9.

11. Verfahren zur Herstellung von RIP SO-6 oder einem Konjugat, umfassend das Konjugat, das an einen Ligand, der zur Bindung an Zellen in der Lage ist, konjugiert ist, wobei das Verfahren die Kultur des transformierten Wirts nach Anspruch 10 und die Isolierung des so produzierten RIP oder Konjugats umfaßt.

12. Pharmazeutische Zusammensetzung, umfassend ein Konjugat, das nach einem Verfahren gemäß Anspruch 11 hergestellt wurde, und einen pharmazeutisch annehmbaren Träger oder Verdünner.

## Patentansprüche (Patentansprüche für folgende(n) Vertragsstaat(en): ES)

1. Verfahren zur Herstellung von ribosomalen inaktivierenden Protein (RIP) SO-6 oder einem Konjugat, umfassend das RIP, das an einen Ligand zur Bindung an Zellen fähigen Liganden konjugiert ist, wobei das Verfahren die Kultur eines Wirtes, der mit einem Vektor, der zur Expression des RIPs oder des Konjugats in der Lage ist, transformiert ist, enthaltend die DNA-Sequenz 
1
24
50
GTCACATCAATCACATTAGATCTAGTAAATCCGACCGCGGTCAATACTCATCTTTTGTG
100
GATAAAATCCGAAACAACGTAAAGGATCCAAACCTGAAATACGGTGGTACCGACATAGCC
150
GTGATAGGCCCACCTTCTAAAGAAAAATTCCTTAGAATTAATTTCCAAAGTTCCCGAGGA
200
ACGGTCTCACTTGGCCTAAAACGCGATAACTTGTATGTGGTCGCGTATCTTGCAATGGAT
250
300
AACACGAATGTTAATCGGGCATATTACTTCAGATCAGAAATTACTTCCGCCGAGTCAACC
350
GCCCTTTTCCCAGAGGCCACAACTGCAAATCAGAAAGCTTTAGAATACACAGAAGATTAT
400
CAGTCGATTGAAAAGAATGCCCAGATAACACAAGGAGATCAAAGTAGAAAAGAACTCGGG
450
TTGGGGATTGACTTACTTTCAACGTCCATGGAAGCAGTGAACAAGAAGGCACGTGTGGTT
500
AAAGACGAAGCTAGATTCCTTCTTATCGCTATTCAGATGACGGCTG
550
AGGCAGCGCGATTTAGGTACATACAAAACTTGGTAATCAAGAACTTTCCCAACAAGTTCAA
600
 
CTCGGAAAACAAAGTGATTCAGTTTGAGGTTAACTGGAAAAAAATTTCTACGGCAATATA
650                                                    700
CGGGGATGCCAAAAACGGCGTGTTTAATAAAGATTATGATTTCGGGTTTGGAAAAGTTAG
                                                  750
GCAGGTGAAGGACTTGCAAATGGGACTCCTTATGTATTTGGCAAACCAAAGTCGTC
 768
AAAC
 und Isolieren des so produzierten RIPs oder Konjugats umfasst.

2. Verfahren nach Anspruch 1, worin der DNA-Sequenz unmittelbar die folgende Signalsequenz vorausgeht:

ATGAAGATATATGTTGTAGCCACAATAGCATGGATCCTGCTTCAATTTTCAGCTTGGACA
ACAACTGATGCG.

3. Verfahren nach Anspruch 1, worin der Vektor die in Fig. 3 der anliegenden Zeichnungen gezeigte DNA-Sequenz enthält.

4. Verfahren nach Anspruch 1, 2, oder 3, worin der Vektor ein Plasmid ist.

5. Vektor nach Anspruch 1, 2 oder 3, worin der Vektor ein viraler Phagen-Vektor ist.

6. Verfahren nach einem der vorhergehenden Ansprüche, das ferner umfaßt die Formulierung des Konjugas mit einem pharmazeutisch annehmbaren Träger oder einem Verdünner unter Herstellung einer pharmazeutisch annehmbaren Zusammensetzung.

7. Verfahren zur Herstellung eines DNA-Moleküls, umfassend die DNA-Sequenz gemäß Anspruch 1, wobei das Verfahren umfaßt:
(i) Isolierung von mRNA aus Blättern von Saponaria officinalis;
(ii) Synthese von cDNA hieraus;
(iii) Einfügen der erhaltenen cDNA in einen Klonierungsvektor unter Erhalt einer cDNA-Bank;
(iv) Untersuchen der cDNA-Bank mit einer markierten DNA-Sonde, die einem Teil der Aminosäuresequenz des RIPs entspricht, zur Lokalisierung eines Klons, der die DNA-Sequenz enthält, und
(v) Isolieren der DNA-Sequenz, die die DNA-Sequenz enthält, aus dem Klon.

## Revendications (Revendications pour l'(les) Etat(s) contractant(s) suivant(s): BE, FR, DE, NL, SE, CH)

1. Séquence d'ADN qui code pour la protéine d'inactivation ribosomique (ribosomal inactivating protein - RIP) SO-6 et qui est :

1                    24                    50
GTCACATCAATCACATTAGATCTAGTAAATCCGACCGCGGTCAATACTCATCTTTTGTG
                                        100
GATAAAATCCGAAACAACGTAAAGGATCCAAACCTGAAATACGGTGGTACCGACATAGCC
                         150
GTGATAGGCCCACCTTCTAAAGAAAAATTCCTTAGAATTAATTTCCAAAGTTCCCGAGGA
                200
ACGGTCTCACTTGGCCTAAAACGCGATAACTTGTATGTGGTCGCGTATCTTGCAATGGAT
     250                                                300
AACACGAATGTTAATCGGGCATATTACTTCAGATCAGAAATTACTTCCGCCGAGTCAACC
                                            350
GCCCTTTTCCCAGAGGCCACAACTGCAAATCAGAAAGCTTTAGAATACACAGAAGATTAT
                                   400
CAGTCGATTGAAAAGAATGCCCAGATAACACAAGGAGATCAAAGTAGAAAAGAACTCGGG
                         450
TTGGGGATTGACTTACTTTCAACGTCCATGGAAGCAGTGAACAAGAAGGCACGTGTGGTT
                500
AAAGACGAAGCTAGATTCCTTCTTATCGCTATTCAGATGACGGCTG
                    550
AGGCAGCGCGATTTAGGTACATACAAAACTTGGTAATCAAGAACTTTCCCAACAAGTTCAA
          600
CTCGGAAAACAAAGTGATTCAGTTTGAGGTTAACTGGAAAAAAATTTCTACGGCAATATA
650                                             700

CGGGGATGCCAAAAACGGCGTGTTTAATAAAGATTATGATTTCGGGTTTGGAAAAGTTAG

750

GCAGGTGAAGGACTTGCAAATGGGACTCCTTATGTATTTGGGCAAACCAAAGTCGTC

768
AAAC.

2. Séquence d'ADN composée de la séquence d'ADN de la revendication 1, immédiatement précédée par la séquence signal suivante :

ATGAAGATATATGTTGTAGCCACAATAGCATGGATCCTGCTTCAATTTTCAGCTTGG
ACAACAACTGATGCG.

3. Séquence d'ADN telle que représentée sur la Figure 3 des dessins annexes.

4. Vecteur de clonage comprenant une séquence d'ADN telle que définie à l'une des revendications précédentes.

5. Vecteur selon la revendication 4, qui est un plasmide.

6. Vecteur selon la revendication 5, qui est un vecteur viral de phage.

7. Vecteur d'expression qui incorpore une séquence d'ADN telle que définie à l'une des revendications 1 à 3 et qui est capable, dans un hôte transformé, d'exprimer ladite RIP.

8. Vecteur selon la revendication 7, qui est un plasmide.

9. Vecteur selon la revendication 7 ou 8, qui est capable, dans un hôte transformé, d'exprimer un conjugué comprenant ladite RIP conjuguée à un ligand capable de se lier aux cellules.

10. Hôte transformé par un vecteur tel que défini à l'une des revendications 7 à 9.

11. Procédé de préparation de la RIP SO-6 ou d'un conjugué comprenant ladite RIP conjuguée à un ligand capable de se lier aux cellules, lequel procédé comprend la culture d'un hôte transformé tel que défini à la revendication 10, et l'isolement de ladite RIP ou dudit conjugué ainsi obtenu.

12. Composition pharmaceutique comprenant un conjugué qui a été obtenu par un procédé tel que défini à la revendication 11 et un support ou diluant pharmaceutiquement acceptable.

## Revendications (Revendications pour l'(les) Etat(s) contractant(s) suivant(s): ES)

1. Procédé de préparation de la protéine d'inactivation ribosomique (ribosomal inactivating protein - RIP) SO-6 ou d'un conjugué comprenant ladite RIP conjuguée à un ligand capable de se lier aux cellules, lequel procédé comprend la culture d'un hôte transformé par un vecteur capable d'exprimer ladite RIP ou ledit conjugué, comprenant la séquence d'ADN : 
1
24
50
GTCACATCAATCACATTAGATCTAGTAAATCCGACCGCGGTCAATACTCATCTTTTGTG
100
GATAAAATCCGAAACAACGTAAAGGATCCAAACCTGAAATACGGTGGTACCGACATAGCC
150
GTGATAGGCCCACCTTCTAAAGAAAAATTCCTTAGAATTAATTTCCAAAGTTCCCGAGGA
200
ACGGTCTCACTTGGCCTAAAACGCGATAACTTGTATGTGGTCGCGTATCTTGCAATGGAT
250
300
AACACGAATGTTAATCGGGCATATTACTTCAGATCAGAAATTACTTCCGCCGAGTCAACC
350
GCCCTTTTCCCAGAGGCCACAACTGCAAATCAGAAAGCTTTAGAATACACAGAAGATTAT
400
CAGTCGATTGAAAAGAATGCCCAGATAACACAAGGAGATCAAAGTAGAAAAGAACTCGGG
450
TTGGGGATTGACTTACTTTCAACGTCCATGGAAGCAGTGAACAAGAAGGCACGTGTGGTT
500
AAAGACGAAGCTAGATTCCTTCTTATCGCTATTCAGATGACGGCTG
550
AGGCAGCGCGATTTAGGTACATACAAAACTTGGTAATCAAGAACTTTCCCAACAAGTTCAA
600
CTCGGAAAACAAAGTGATTCAGTTTGAGGTTAACTGGAAAAAAATTTCTACGGCAATATA
650
700
CGGGGATGCCAAAAACGGCGTGTTTAATAAAGATTATGATTTCGGGTTTGGAAAAGTTAG
750
GCAGGTGAAGGACTTGCAAATGGGACTCCTTATGTATTTGGGCAAACCAAAGTCGTC
768
AAAC
 et l'isolement de ladite RIP ou dudit conjugué ainsi obtenu.

2. Procédé selon la revendication 1, dans lequel ladite séquence d'ADN est immédiatement précédée par la séquence signal suivante :

ATGAAGATATATGTTGTAGCCACAATAGCATGGATCCTGCTTCAATTTTCAGCTTGG
ACAACAACTGATGCG.

3. Procédé selon la revendication 1, dans lequel le vecteur comprend la séquence d'ADN représentée sur la Figure 3 des dessins annexés.

4. Procédé selon la revendication 1, 2 ou 3, dans lequel ledit vecteur est un plasmide.

5. Procédé selon la revendication 1, 2 ou 3, dans lequel ledit vecteur est un vecteur viral de phage.

6. Procédé selon l'une des revendications précédentes, qui comprend en outre la formulation dudit conjugué avec un support ou diluant pharmaceutiquement acceptable pour produire une composition pharmaceutiquement acceptable.

7. Procédé de production d'une molécule d'ADN comprenant la séquence d'ADN telle que définie à la revendication 1, lequel procédé comprend :
(i) l'isolement d'ARNm à partir des feuilles de Saponaria officinalis ;
(ii) la synthèse de l'ADNc à partir de celui-ci ;
(iii) l'insertion de l'ADNc résultant dans un vecteur de clonage afin d'obtenir une bibliothèque d'ADNc ;
(iv) le sondage de la bibliothèque d'ADNc avec une sonde à ADN marqué correspondant à une fraction de la séquence d'acides aminés de ladite RIP, pour localiser un clone comprenant ladite séquence d'ADN précitée ; et
(v) l'isolement d'une séquence d'ADN comprenant ladite séquence d'ADN à partir du clone.
